# EUROPEAN PATENT APPLICATION

(11) **EP 1 647 273 A1**
(43) Date of publication of application: **19.04.2006**
(21) Application number: 04746179.3
(22) Date of filing: 21.06.2004
(51) Int. Cl.: A61K 31/4415, A61K 47/36, A61K 47/38, A61P 3/02, A61P 27/02

(54) **OPHTHALMIC COMPOSITION**

(30) Priority: 20.06.2003 JP 2003176965
(71) Applicant: KOBAYASHI PHARMACEUTICAL CO. LTD., Osaka-shi, Osaka 5418507 (JP)
(72) Inventor: MATSUI, Yuka, c/o Kobayashi Pharm. Co., Ltd R&D CO, Ibaraki-shi,Osaka 567-0057 (JP)
(74) Representative: Pett, Christopher Phineas
(86) International application number: PCT/JP2004/008710
(87) International publication number: WO 2004/112789

(57) **Abstract**

An ophthalmic composition is described which is prepared by incorporating pyridoxine hydrochloride in combination with chondroitin sulfate and a cellulosic polymer. The composition exhibits reduced or eliminates eye irritation attributable to pyridoxine hydrochloride.

## Description

### [TECHNICAL FIELD]

The present invention relates to an ophthalmic composition. More particularly, of an ophthalmic composition containing pyridoxine hydrochloride, the present invention relates to the ophthalmic composition which exhibits the reduced irritation to eyes. Furthermore, the present invention relates to a process for alleviating irritation to eyes with an ophthalmic composition including pyridoxine hydrochloride.

### [BACKGROUND ART]

In the development of pharmaceutical products for use in the ophthalmologic field, irritations to the ophthalmic mucosa and discomfort have to be always considered in addition to the medical effect thereof. Hence, with regard to various types of active components used in the conventional ophthalmic compositions, some processes for removing or alleviating and moderating the irritation have been proposed.

For example, Prior Art 1 discloses a process for moderating with cyclosporine irritations to mucosa of eyes, nose or the like due to cetirizine to be acted as an antiallergic agent; Prior Art 2 discloses a process for moderating irritations to eyes with 2-(2-fluoro-4-biphenylyl)propionic acid which is used as an anti-inflammatory agent by blending one or two or more of polyvinyl alcohol, methyl cellulose, carboxymethyl cellulose, hydroxyethyl cellulose, hydroxypropyl methyl cellulose and sodium chondroitin sulfate in an amount of 0.01 to 2% to form a composition with the pH 5-8 so adjusted; Prior Arts 3 and 4 disclose a process for moderating irritations to eyes with 2-acetyl-1-(2-hydroxy-8-isopropylaminopropoxy)benzofuran which is used as an intraocular pressure decreasing agent or a therapeutic agent for glaucoma by blending (A) 0.001 to 0.1% benzalkonium chloride or benzethonium chloride, and (B) at least one compound of polyvinyl alcohol, methyl cellulose, carboxymethyl cellulose and hydroxyethyl cellulose in an amount of 0.02 to 2 w/v%, or hydroxypropyl methyl cellulose in an amount of 0.01 to 1 w/v% to form a composition with the pH 5-8 so adjusted; and, Prior Art 5 disocloses a process for moderating irritations to eyes with lower alcohol such as ethanol which is used as a refrigerant by blending a saccharide such as mannitol, xylitol, glucose and maltose.

Accordingly, variable types of irritating components are available, and under current circumstances, processes for removing or alleviating and moderating the wide variety of different irritations have been studied and proposed depending on the type thereof.
Prior Art 1; Japanese Patent Provisional Publication No. 6-239748.
Prior Art 2; Japanese Patent Provisional Publication No. 57-102817.
Prior Art 3; Japanese Patent Provisional Publication No. 56-39013.
Prior Art 4; Japanese Patent Provisional Publication No. 57-16817.
Prior Art 5; Japanese Patent Provisional Publication No. 2001-261578.

### [DISCLOSURE OF THE INVENTION]

### [PROBLEMS TO BE SOLVED BY THE INVENTION]

An object of the present invention is to provide a process for alleviating an irritation or discomfort to eyes with pyridoxine hydrochloride which has often been used in ophthalmic-pharmaceutical compositions to expect the moderating effects of asthenopia, and an ophthalmic composition which alleviates an irritation to eyes according to the process.

### [MEANS TO SOLVE THE PROBLEMS]

The present inventor investigated to develop an ophthalmic composition to moderate asthenopia, and discovered that an ophthalmic composition containing pyridoxine hydrochloride as an active component to moderate asthenopia may cause an unpleasant irritation to the ophthalmic mucosa. Hence, as a consequence of elaborate investigations for eliminating such problems and for obtaining a desired ophthalmic composition as described above, it was found that preparation of an ophthalmic composition containing the combined components of chondroitin sulfate salt and cellulose based polymer compound, in addition to pyridoxine hydrochloride, enables an ophthalmic composition to moderate asthenopia without unpleasant irritation through alleviating or removing irritation to be generated from pyridoxine hydrochloride. Furthermore, the effect of alleviating or removing the unpleasant irritation allows using a large amount of pyridoxine hydrochloride, thereby, an ophthalmic composition may then moderate asthenopia remarkably. The present invention was accomplished based on such investigation results.

Accordingly, merits of the present invention are as follows.
(1) An ophthalmic composition which comprises pyridoxine hydrochloride, chondroitin sulfate salt and cellulose based polymer compound.
(2) The ophthalmic composition according to the item (1) wherein said cellulose based polymer compound is at least one compound selected from hydroxyethyl cellulose, methyl cellulose, and hydroxypropyl methyl cellulose.
(3) A process for alleviating an irritation to eyes with an ophthalmic composition containing pyridoxine hydrochloride, the process comprises blending chondroitin sulfate salt and cellulose based polymer compound together with an ophthalmic composition containing pyridoxine hydrochloride.
(4) The process for alleviating an irritation according to the item (3) wherein said cellulose based polymer compound is at least one compound selected from hydroxyethyl cellulose, methyl cellulose, and hydroxypropyl methyl cellulose.

### [EFFECTS OF THE INVENTION]

The ophthalmic composition of the present invention has an effect to moderate asthenopia by pyridoxine hydrochloride used as an active component thereof, and an irritation to eyes with pyridoxine hydrochloride is also alleviated or removed by the combined components of chondroitin sulfate salt and cellulose based polymer compound. Therefore, according to the present invention, an ophthalmic composition can be used without unacceptable sense, and an effect to moderate asthenopia can be offered. Moreover, according to the present invention, a process for removing or alleviating an irritation to eyes with pyridoxine hydrochloride can be provided.

### [BEST MODE FOR CARRYING OUT THE INVENTION]

### (1) Ophthalmic Composition

The ophthalmic composition of the present invention uses pyridoxine hydrochloride as an active component, and the combined components of chondroitin sulfate salt and cellulose based polymer allow to accompolish the merits of the present invention to provide an ophthalmic composition composition which can be used without unacceptable sense and can offer an improved effect to moderate asthenopia due to pyridoxine hydrochloride, through alleviation or removal of the irritation resulting from the aforementioned pyridoxine hydrochloride.

Chondroitin sulfate salt to be used in the present invention is not particularly limited as long as it is a pharmaceutically acceptable salt of chondroitin sulfate, but may be usually sodium chondroitin sulfate. An amount of the chondroitin sulfate salt to be blended into the ophthalmic composition is not particularly limited as long as the merit of the present invention is accomplished, but illustrative range thereof may be 0.001 w/v% or more, preferably 0.001 to 0.5 w/v%, and more preferably 0.005 to 0.5 w/v% in 100 w/v% of the ophthalmic composition. Also, exemplary ratio to pyridoxine hydrochloride to be blended into the ophthalmic composition may be 0.01 to 2,000 parts by weight, preferably 0.05 to 2,000 parts by weight, and more preferably 0.05 to 500 parts by weight per 1 part by weight of pyridoxine hydrochloride.

Furthermore, specific examples of the cellulose based polymer compound which may be used in the present invention include alkyl cellulose such as methyl cellulose, ethyl cellulose and carboxymethyl cellulose; hydroxyalkyl cellulose such as hydroxyethyl cellulose, hydroxyethyl methyl cellulose, hydroxypropyl cellulose and hydroxypropyl methyl cellulose. Preferably, it may be methyl cellulose, hydroxypropyl methyl cellulose, or hydroxyethyl cellulose, and more preferably, methyl cellulose or hydroxypropyl methyl cellulose. These may be used alone, or in optional combination of two or more of them.

An amount of the blended cellulose based polymer compound to be used in the ophthalmic composition is not particularly limited as long as the merit of the present invention is accomplished. In general, the amount thereof can not be determined regularly, because it may vary depending on the type of the cellulose based polymer compound as actually used, but can be selected and adjusted ad libitum to fall within the range of 0.01 to 10 w/v%, preferably 0.01 to 5 w/v%, and more preferably 0.05 to 5 w/v% in 100 w/v% of the ophthalmic composition as a reference. Also, exemplary ratio to pyridoxine hydrochloride blended into the ophthalmic composition may be 0.1 to 10,000 parts by weight, preferably 0.1 to 5,000 parts by weight, and more preferably 0.1 to 200 parts by weight per 1 part by weight of pyridoxine hydrochloride. Moreover, it is desired to adjust the ratio of the cellulose based polymer compound per 1 part by weight of the chondroitin sulfate salt blended into the ophthalmic composition appropriately to be 0.02 to 10,000 parts by weight, preferably 0.02 to 5,000 parts by weight, and more preferably 0.02 to 1,000 parts by weight.

The concentration of pyridoxine hydrochloride in the ophthalmic composition of the present invention may vary widely depending on the particular use of the composition (either pharmaceutical use or the other use) and extent of the asthenopia to be ameliorated, but may be usually 0.001 w/v% or more, preferably 0.001 to 1 w/v%, and more preferably 0.001 to 0.1 w/v%.

The ophthalmic composition of the present invention is preferably adjusted to the pH range which is generally acceptable for ophthalmic applications. Specifically, pH may fall within the range of from 4 to 9, preferably 5 to 8, and more preferably 5.5 to 8.

Furthermore, the ophthalmic composition of the present invention is preferably adjusted to the osmotic pressure range which is generally acceptable for ophthalmic applications. Specifically, it is preferably adjusted to be a pressure ratio falling within the range of 0.5 to 5, and more preferably within the range of the pressure ratio of 0.8 to 2. For adjusting the osmotic pressure, for example, any method usually adopted in preparation of eye drops can be used in a similar manner.

Besides pyridoxine hydrochloride, any component to be acted to moderate asthenopia may be blended in the ophthalmic composition of the present invention as long as the merit of the present invention is not impaired. Also, other pharmaceutically effective components commonly used in ophthalmologic field may also be blended ad libitum.

Such pharmaceutically effective components are not limited, and illustrative examples thereof include decongestants (e.g., naphazoline hydrochloride, tetrahydrozoline hydrochloride, phenylephrine hydrochloride, epinephrine hydrochloride and the like), antiphlogistic, astringent drugs (e.g., neostigmine methylsulfate, ε -amino caproic acid, allantoin, berberine chloride, zinc sulfate, lysozyme chloride, sodium azulene sulfonate, dipotassium glycyrrhizinate and the like), antiallergic agents (diphenhydramine hydrochloride, isopenzyl hydrochloride, chlorpheniramine maleate, sodium cromoglycate and the like), vitamins other than pyridoxine hydrochloride (e.g., vitamin B₂, vitamin B₁₂, vitamin A, vitamin E, calcium pantothenate and the like), amino acids (potassium L-aspartate, magnesium L-aspartate, aminoethylsulfonic acid and the like), sulfa drugs (e.g., sulfamethoxazole, sulfisoxazole, sulfisomidine and the like), bacteriocides (sulfur, isopropylmethyl phenol, hinokithiol and the like), topical anesthetics (lidocaine, lidocaine hydrochloride, procaine hydrochloride, dibucaine hydrochloride and the like), inorganic salts (e.g., potassium chloride, sodium chloride, sodium bicarbonate and the like), thickening agents (polyvinyl alcohol, polyvinyl pyrrolidone, carboxymethyl cellulose, hyaluronic acid, glucose and the like), but not limited thereto.

A variety of additives (e.g., solubilization auxiliary agents, isotonizing agents, stabilizing agents, chelating agents, pH adjusting agents, refrigerants, preservatives, and thickening agents) as well as a carrier (e.g., buffer agents, and ointment bases) which may be generally used in ophthalmic compositions can also be blended, in addition to the aforementioned essential components, into the ophthalmic composition of the present invention in the range not to compromise the merit of the present invention.

Illustrative examples of the solubilization auxiliary agent include polyethylene glycol, propylene glycol and the like; isotonizing agent include sodium chloride, potassium chloride, sorbitol, mannitol, glycerin and the like; stabilizing agent include sodium edetate, cyclodextrin, sulfite, citric acid or salts thereof, and the like; the chelating agent include sodium edetate, sodium citrate and the like; pH adjusting agent include hydrochloric acid, citric acid or salts thereof, boric acid or salts thereof, phosphoric acid or salts thereof, acetic acid or salts thereof, tartaric acid or salts thereof, sodium hydroxide or potassium hydroxide, and the like; refrigerant include monoterpenoid compounds such as menthol, camphor, borneol, geraniol, cineol, limonene and eugenol, or peppermint oil, bergamot oil, eucalyptus oil, fennel oil, cool mint, and the like; examples of the preservative include paraoxybenzoic acid esters, benzalkonium chloride, chlorobutanol and the like; and moreover, thickening agent include polyvinyl alcohol, polyvinylpyrrolidone, carboxymethyl cellulose, hyaluronic acid, glucose and the like.

Furthermore, illustrative examples of the buffer agent include phosphoric acid or salts thereof (e.g., sodium monohydrogen phosphate and the like), boric acid or salts thereof (e.g., borax and the like), citric acid or salts thereof (e.g., sodium citrate and the like), tartaric acid or salts thereof (e.g., sodium tartrate and the like), gluconic acid or salts thereof (e.g., sodium gluconate and the like), acetic acid or salts thereof (e.g., sodium acetate and the like), various amino acids, and the like.

Moreover, illustrative examples of the base for use in the ophthalmic ointment include, for example, white petrolatum, liquid paraffin, carboxymethyl cellulose, macrogol, carboxyvinyl polymer, and the like.

These compositions can be of any formulation generally used in ophthalmic compositions. Examples of such formulation include, for example, aqueous solutions, suspensions, emulsions, gelatinous materials, ointments and the like. Also, the dosage form is not particularly limited, but any form such as eye drops (including those for contact lenses), ophthalmic ointments, or eye lotions may be permitted. Furthermore, a solid type formulation prepared before use may be permitted which is obtained by solidifying the composition of the present invention by a process such as freeze-drying followed by forming a solid formulation like powder, granule or tablet form to be used after dissolution or the like in purified water upon use.

The process for preparing the ophthalmic composition of the present invention is not particularly limited, but may be prepared according to common procedures for ophthalmic compositions. For example, the composition can be prepared by dissolving each component described above in water such as sterile purified water or ion exchanged water, or in a mixed solvent of the water and a lower alcohol such as ethanol, and thereafter, pH or osmotic pressure of the composition is adjusted appropriately with a pH adjusting agent, an isotonizing agent or the like.

Preferrably, the ophthalmic composition of the present invention is administered, for example, into an adult in the form of an eye drops by dropping to eye one to few drop(s) per once approximately 3 to 6 times per day.

### [EXAMPLES]

Hereinafter, the present invention will be illustrated in detail by way of Example and Relative, but the present invention is not limited anyhow by the disclosure thereof.

### Examples 1-2, Relatives 1-6 and Control

Eye drops made from the prescription shown in Table 1 were prepared (Examples 1-2, Relatives 1-6, Control), and evaluated on the irritation when they were dropped in eyes. The viscosity of the eye drops is indicated as a value determined with B type viscometer at 20°C.

Irritation to eyes was evaluated with sensory test wherein ten persons of adult men and women were participated. Each person rated irritation according to the following standard for eye drops of each prescription and evaluated it on the basis of the total points.

**<Evaluation on Moderation of Irritation>**

| | | |
|---|---|---|
| No irritation experienced at all; | 10 | points |
| Irritation not experienced well; | 5 | points |
| Undecidable; | 0 | point |
| Irritation somewhat experienced; | -5 | points |
| Irritation experienced; | -10 | points |

The results are also shown in Table 1.

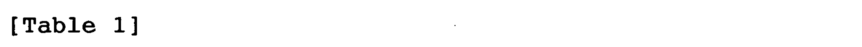

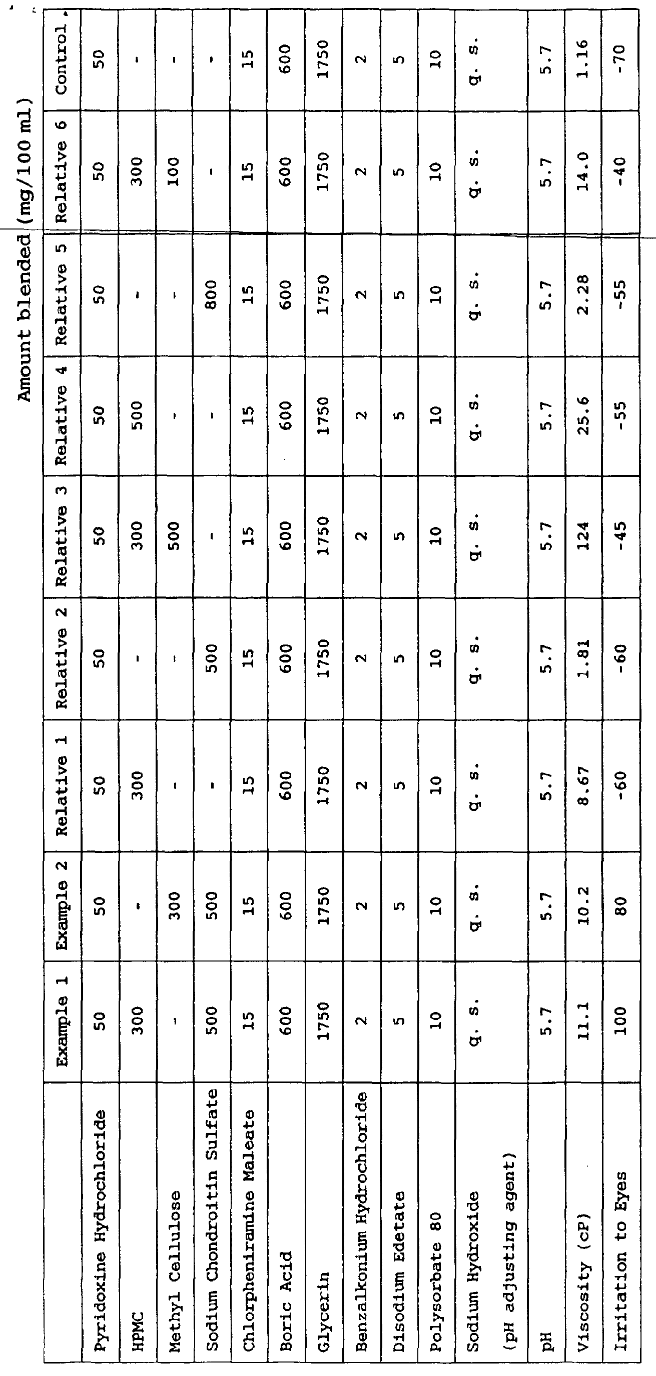

In the Table, "HPMC" means hydroxypropyl methyl cellulose. As shown in Table 1, irritation to eyes with pyridoxine hydrochloride (Control) was revealed to be markedly alleviated or removed by using the combined components of the cellulose based polymer compound and sodium chondroitin sulfate (Examples 1 and 2). Furthermore, this effect was not exerted unless both the cellulose based polymer compound and sodium chondroitin sulfate were used, while the cellulose based polymer compound alone (Relatives 1 and 4) or combination thereof (Relatives 3 and 6), and sodium chondroitin sulfate alone (Relatives 2 and 5) exhibited no effect at all. Moreover, from the results of Relatives 1 and 4, Relatives 2 and 5, and Relatives 3 and 6 shown in Table 1, it was elucidated that the viscosity of the ophthalmic composition does not affect the moderation of irritation to eyes with pyridoxine hydrochloride.

### Example 3

An ophthalmic composition in the form of ointment was prepared according to the following prescription.

| | | |
|---|---|---|
| Pyridoxine Hydrochloride | 100 | mg |
| HPMC | 500 | |
| Sodium Chondroitin Sulfate | 500 | |
| Sodium Cromoglycate | 1,000 | |
| Carboxymethyl Cellulose | 4,000 | |
| Glycerin | 2,660 | |
| Benzalkonium Chloride | 5 | |
| Polysorbate 80 | 20 | |
| Sodium hydroxide | q.s. | (pH 5.7) |
| Sterile Purified Water | Residual amount | |
| Total | 100 | mL |

### Example 4

An ophthalmic solution was prepared according to the following prescription.

| | | |
|---|---|---|
| Pyridoxine Hydrochloride | 5 | mg |
| HPMC | 1,000 | |
| Sodium Chondroitin Sulfate | 50 | |
| Disodium Glycyrrhizinate | 5 | |
| Boric Acid | 1,000 | |
| Benzalkonium Chloride | 5 | |
| Disodium Edetate | 5 | |
| Polysorbate 80 | 20 | |
| Sodium Hydroxide | q.s. | (pH 5.7) |
| Sterile Purified Water | Residual amount | |
| Total | 100 | mL |

### Example 5

An eye drop was prepared according to the following prescription.

| | | |
|---|---|---|
| Pyridoxine Hydrochloride | 50 | mg |
| HPMC | 300 | |
| Sodium Chondroitin Sulfate | 500 | |
| Chlorpheniramine Maleate | 15 | |
| Boric Acid | 600 | |
| Glycerin | 1,750 | |
| Benzalkonium Chloride | 2 | |
| Disodium Edetate | 5 | |
| Polysorbate 80 | 10 | |
| Sodium Hydroxide | q.s. | (pH 5.7) |
| Sterile Purified Water | Residual amount | |
| Total | 100 | mL |

## Claims

1. An ophthalmic composition which comprises pyridoxine hydrochloride, chondroitin sulfate salt and cellulose based polymer compound.

2. The ophthalmic composition according to claim 1 wherein said cellulose based polymer compound is at least one compound selected from hydroxyethyl cellulose, methyl cellulose, and hydroxypropyl methyl cellulose.

3. A process for alleviating an irritation to eyes with an ophthalmic composition containing pyridoxine hydrochloride, the process comprises blending a chondroitin sulfate salt and cellulose based polymer compound together with an ophthalmic composition containing pyridoxine hydrochloride.

4. The process for alleviating an irritation according to claim 3, wherein said cellulose based polymer compound is at least one compound selected from hydroxyethyl cellulose, methyl cellulose, and hydroxypropyl methyl cellulose.
